# EUROPEAN PATENT SPECIFICATION

(11) **EP 2 873 372 B1**
(45) Date of publication and mention of the grant of the patent: **02.10.2019**
(21) Application number: 14192446.4
(22) Date of filing: 10.11.2014
(51) Int. Cl.: A61B 10/02, A61B 17/34

(54) **Biopsy needle system for MR-guided biopsy**
Biopsienadelsystem für MR-geführte Biopsie
Système d'aiguille de biopsie pour biopsie guidée par RM

(30) Priority: 13.11.2013 DE 102013112471
(43) Date of publication of application: 20.05.2015
(73) Proprietor: Lumiani, Agron, 33617 Bielefeld (DE)
(72) Inventor: Lumiani, Agron, 33617 Bielefeld (DE)
(74) Representative: Wittmann, Günther

(56) References cited:
- WO-A1-96/14023
- WO-A1-97/07746
- WO-A1-2005/104973
- US-A1- 2007 260 267
- US-A1- 2008 139 928

## Description

The present invention relates to a biopsy needle for the MR-guided biopsy for application in a magnet resonance tomograph of arbitrary magnetic field strength, particularly having a magnetic field strenght of 3 Tesla and more.

During MR-guided biopsy a biopsy needle is moved by a physician in the direction of the lesion. In predetermined intervals it is verified by imaging methods using a magnet resonance tomograph that the biopsy needle is moved to the lesion. If necessary a physician can apply corrections to ensure that the needle is moved to the lesion.

In a magnet resonance tomograph signals of hydrogen atoms are generated under the influence of magnetic fields and dynamic magnetic fields, respectively in interaction with RF-radiation. The operation of a magnet resonance tomograph is known to the person skilled in the art and does not have to be explained in more detail herein.

Early magnet resonance tomographs had a magnetic field of 0.2 Tesla. Successively magnet resonance tomographs having a higher magnetic field strength became commercially available. Magnet resonance tomographs having a magnetic field strength of 1.0 Tesla and 1.5 Tesla have been introduced later and were accepted by the market and are worldwide available.

Magnet resonance tomographs having a magnetic field strength of 1.0 Tesla and 1.5 Tesla are increasingly replaced by magnet resonance tomographs having a field strength of 3 Tesla. Magnet resonance tomographs having a magnet field strength of 7 Tesla are also available.

Needles currently approved for MR-guided biopsy are made of titanium or comprise titanium coated portions. Such needles are MRT compatible up to a magnetic field strength of 1.5 Tesla. However, artifacts are caused in the region of the needles by these needles and a magnetic field strength of 1.5 Tesla.

A biopsy needle of the prior art for the MR-guided prostate biopsy comprises a hollow needle also termed coaxial portion. The biopsy needle further comprises a so-called mandrin having a sharp tip, which is introduced in use into the hollow needle and is used finally for punction. Both the hollow needle and the mandrin are as mentioned before made the by titanium or are titanium coated. Titanium is a non ferromagnetic material.

During MR-guided biopsy the patient is lying on the table of the magnet resonance tomograph. At the patient so-called local coils may be arranged, for example. Before punction the patient is moved into the magnet resonance tomograph and imaging is performed by means of RF signals and gradients. After imaging the patient is moved out of the magnet resonance tomograph, wherein the patient keeps on lying on the table of the magnet resonance tomograph. The portion to be punctured is anesthetized locally and the skin is cut (incision) such that the hollow needle can enter the patient.

The physician introduces the hollow needle and the mandrin into the patient, wherein the patient is located outside of the magnet resonance tomograph.

When the physician has introduced the hollow needle and the mandrin by a predetermined distance into the patient, the physician can carry out a further imaging. Hereby, the patient is moved into the magnet resonance tomograph and in the region of its uniform magnet field, whereafter imaging is carried out by RF-signals and gradients signals.

Subsequently the physician can introduce the hollow needle and the mandrin further into the patient. The sequence of imaging and introducing the hollow needle including the mandrin may be repeated until the hollow needle has arrived at the lesion and the portion to be punctured, respectively. As soon as the hollow needle is positioned at the region to be punctured, which may be verified by imaging, the mandrin is removed and the hollow needle is fixed, for example by adhesive plaster. Subsequently, a biopsy needle is introduced into the hollow needle for removing tissue.

The MR-guided biopsy is known to the person skilled in the art and is not explained in further detail for the sake of brevity.

Magnet resonance tomograph having a magnetic field strength of 1.5 Tesla use radiofrequency signals having 63.87 MHz. Magnet resonance tomographs having a magnetic field strength of 3 Tesla use a radiofrequency signal having 127.73 MHz corresponding to a wave length of approximately 2.3 m. Such radiofrequency signal can heat a hollow needle and/or a mandrin, particularly if it comprises a length of approximately 16 cm to approximately 20 cm or more, which is inconvenient for the patient and may course in the worse case a local burn of the patient.

It is an object of the present invention to provide an improved biopsy needle system for MR-guided biopsy.

The object of the invention is achieved by a biopsy needle system according to claim 1. The dependent claims describe preferred embodiments.

A cylindrical portion adapted to be introduced into a patient of an inventive hollow needle of a biopsy needle system is formed by a non ferromagnetic and/or non-metallic material. Thereby, it may be ensured that the hollow needle is not heated by the high-frequency signal. Therefore, during MR-guided biopsy no unpleasant side effects for the patient occur. Burns may be avoided. Further, artifacts may be avoided.

The cylindrical portion of the hollow needle can be made of plastics, carbon, carbon fiber and/or ceramics. Particularly suited are hard plastics.

WO 96/14023 discloses an apparatus for examining tissue having a hollow cannula, in which a coaxial cannula and a needle device may be inserted. The hollow cannula is made of plastics. The coaxial cannula may be filled with a contrast agent. Further, the hollow cannula comprises a sharp tip for piercing tissue.

US 2007/0260267 A1 discloses a reference structure on which a cannula may be mounted. The reference structure defines a position along the X and Y axes. A cannula protrudes into the body of a patient. By the cannula fluids may be inserted into or removed from the patients. An introducer stylet may be introduced into the cannula. The introducer stylet exits from the distal opening of the cannula into the patient's body and creates a pathway to the target tissue. After the introducer stylet is removed from the outer cannula, a target confirmation device may be inserted into the patient's body through the port created by the outer cannula.

US 2008/0139928 A1 discloses an obturator with an elongated shaft, a proximal end, a substantially closed distal end and an MRI detectable distal shaft portion, which does not interfere with magnetic resonance imaging of the tissue proximate thereto.

WO 97/07746 A1 discloses a medical needle made of non-metallic, non-magnetic materials such that medical interventional procedures requiring needle access to people, animals or isolated tissues can be performed in a MRI scanner without significant artefact or image distortions.

It is an object of the present invention to provide an improved biopsy needle system for MR-guided biopsy.

The object of the invention is achieved by a biopsy needle system according to claim 1. The dependent claims describe preferred embodiments.

A cylindrical portion adapted to be introduced into a patient of an inventive hollow needle of a biopsy needle system is formed by a non ferromagnetic and/or non-metallic material.

Thereby, it may be ensured that the hollow needle is not heated by the high-frequency signal. Therefore, during MR-guided biopsy no unpleasant side effects for the patient occur. Burns may be avoided. Further, artifacts may be avoided.

The cylindrical portion of the hollow needle can be made of plastics, carbon, carbon fiber and/or ceramics. Particularly suited are hard plastics.

The invention also relates to a biopsy needle system comprising a hollow needle, a first mandrin having a sharp tip and an imaging mandrin. The first mandrin comprising the sharp tip may be introduced into the hollow needle such that the tip and an opposing end of the mandrin extend from opposing ends of the hollow needle, when the hollow needle is located in a patient or is introduced into the patient, wherein the sharp tip at the distal and is adapted to pierce, perforate and/or puncture tissue. The imaging mandrin comprises a second material and may be introduced into the hollow needle, if the hollow needle is located in a patient. The second material is a material that may be imaged appropriately to the using MR-imaging.

By imaging using a T1-weighted sequence a contrast agent comprising gadolinium may be imaged a appropriately. By a T1-weighted sequence also oil and fish oil may be appropriately imaged by a somewhat lower intensity. By imaging using a T2-weighted sequence oil, fish oil and water can be appropriately imaged. By a T2-weighted sequence also contrast agent comprising gadolinium can be imaged appropriately by a slightly lower intensity.

Using MR-imaging oil and fish oil can be imaged by a series of a T2-weighted sequence, a T1-weighted sequence and T1-FS weighted sequence, wherein FS is the appreciation for "Fat Subpression" and the order of the sequence is arbitrary. The contrast agent may be imaged by a series of a T1-weighted sequence, a T1-FS weighted sequence and a T2-weighted sequence, wherein the order of the sequence is also arbitrary.

In the context of the present invention appropriately imaged by MR-imaging means that the number and/or the density of the hydrogen atoms and/or of the protons in the second material is at least 5%, preferably at least 10%, preferably at least 15%, more preferred at least 25%, more preferred at least 30%, most preferred at least 50% higher or lower as the nuclear spin signal of the tissue around the hollow needle due to the hydrogen atoms and/or the protons and/or as the nuclear spin signal of the body tissue due to the hydrogen atoms and/or the protons therein. The body tissues may comprise soft tissue, organ tissue, skin tissue and/or nerve tissue.

In the context of the present invention appropriately imaged by MR-imaging means that the nuclear spin signal due to gadolinium in the second material is at least 5%, preferably at least 10%, more preferred at least 15%, more preferred at least 25%, more preferred at least 30%, most preferred at least 50% higher as the nuclear spin signal of the tissue around the hydrogen atoms and/or the protons around the hollow needle and/or as the nuclear spin signal of the body tissue due to the hydrogen atoms and/or the protons in the body tissue.

The hollow needle comprises a cylindrical portion adapted to be introduced into a patient. The cylindrical portion comprises a first material which is a non ferromagnetic and/or a non-metallic material, such as plastics, carbon, carbon tissue and/or ceramics. The imaging mandrin comprises plastics. A cylindrical portion of the imaging mandrin may comprise plastics, wherein the cylindrical portion is adapted to be introduced into the hollow needle.

The imaging mandrin and the cylindrical portion respectively may comprise a cavity in which a liquid and/or a gel as a second exemplary material may be located. The liquid may comprise oil, fish oil, water and/or contrast agent, for example. The imaging mandrin comprises no sharp tip at the distal end and introduced into the patient and hollow needle. The distal end of imaging mandrin may be blunt.

The invention is now described in more detail and non limiting under reference to the attached drawings showing an exemplary embodiment of the invention wherein:
Figure 1 is a top view on the inventive hollow needle;
Figure 2 is a top view of a mandrin; and

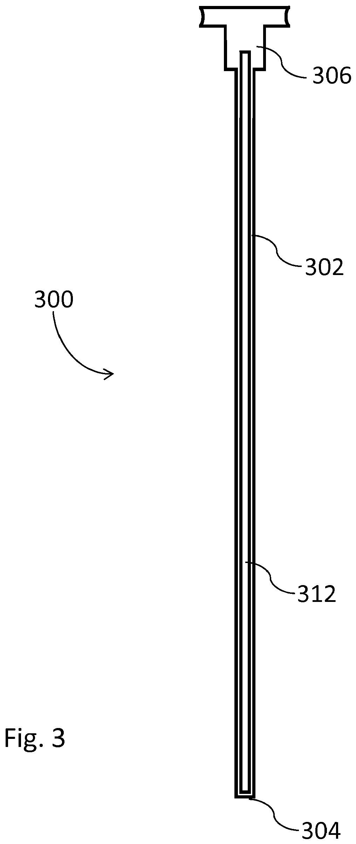

Figure 3 shows an imaging mandrin.

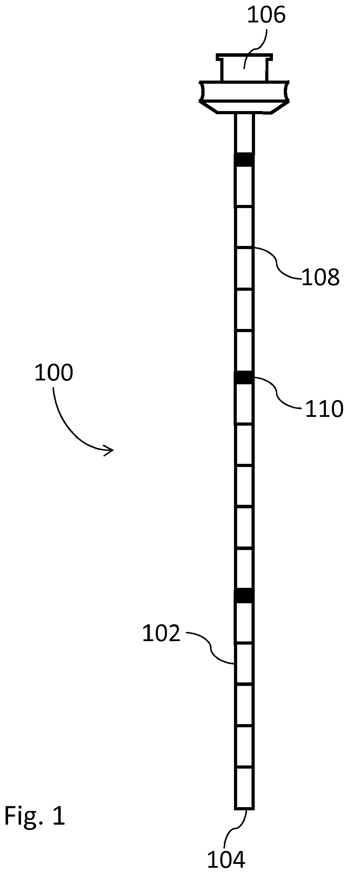

Figure 1 shows a top view of a hollow needle 100 according to the present invention also termed cannula. The hollow needle 100 comprises a cylindrical portion 102, whose interior is hollow, a distal opening 104 to be introduced into the patient and a proximal grip portion 106 protruding in use from the patient. The cross-section of the cylindrical portion 102 is circular and the diameter of the cylindrical portion ranges in the field of prostate biopsy from approximately 1.5 mm to approximately 2 mm, for example. The length of the hollow needle 100 can range in the field of prostate biopsy from approximately 16 cm to approximately 20 cm or more, for example. It is to be understood that a hollow needle for biopsy of other organs may have different dimensions. The hollow needle may be manufactured from a non ferromagnetic and/or a non-metallic material. Particularly the cylindrical portion 102 can be made of a non ferromagnetic material and/or a non-metallic material. Thereby, it can be assured that the hollow needle and particularly the cylindrical portion 102 do not function as an antenna and that the cylindrical portion 102 of the hollow needle 100 is not heated. Thereby, undesired side effects of the MR-imaging due to heating the cylindrical portion 102 up to burns of the patient are avoided. Preferably the hollow needle 100 and particularly the cylindrical portion 102 are made of plastics, carbon, carbon fiber and/or ceramics.

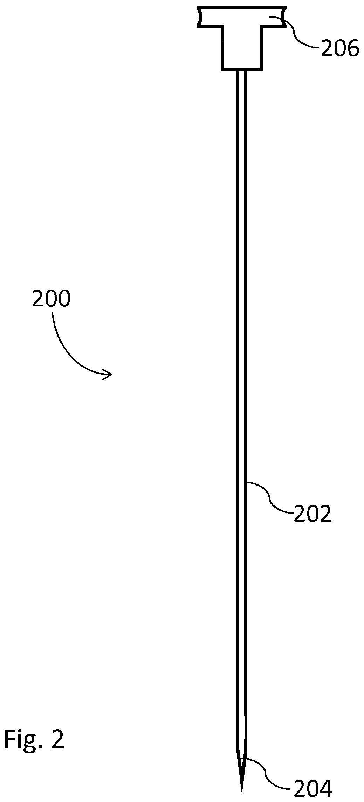

Figure 2 shows a first mandrin used for biopsy and for introducing the hollow needle 100 in the body of a patient. The first mandrin 200 comprises a cylindrical portion 202, wherein on the distal end a sharp tip 204 and on its proximal end 206 a grip is located. The mandrin comprises for example in the field of prostate biopsy a length of approximately 16 cm to approximately 20 cm or more. The cylindrical portion 202 of the first mandrin has for example in the field of prostate biopsy a diameter of approximately 1.5 cm to approximately 2 cm. It is to be understood that a first mandrin for biopsy of other organs may have different dimensions. The cylindrical portion 202 and the tip 204 may be made of titanium or coated by titanium. Such mandrins are certified for biopsy and physicians have experience in use of such mandrins. Accordingly, the actual punction may be executed as used by physicians.

The physician may control introducing the hollow needle 100 and the first mandrin 200 by the proximal grip 106 of the hollow needle 100 and of the proximal grip 206 of the first mandrin.

At the cylindrical portion 102 of the hollow needle 100 marks having a lower subdivision and marks 110 having a larger subdivision are arranged. A plurality of marks 108 having the lower subdivision coincide with a mark 110 having a larger subdivision. The marks 108 having a smaller subdivision may be spaced apart from the next mark 108 having the lower subdivision by 1 cm. A mark 110 having the larger subdivision may be spaced apart 5 cm from the next mark having the larger subdivision 110. By means of the marks 108 having the lower subdivisions and the marks 110 having the larger subdivisions the physician can verify how deep the hollow needle 100 and the cylindrical portion 102, respectively of the hollow needle 100 have been introduced into the body.

The physician introduces the hollow needle 100 with inserted first mandrin 200, wherein the tip 204 of the first mandrin 200 projects from the distal opening 104 of the hollow needle 100, for a predetermined distance into the patient. Thereafter, the physician may verify using MR-imaging, whether the hollow needle 100 has been introduced in the correct direction. Thereby, imaging by a magnet resonance tomograph is carried out.

Since the first mandrin 200 comprises titanium or a titanium coating artifacts may arise and/or the first mandrin 200 may be heated, for example heated such that a burn of a patient occurs, if a magnet resonance tomograph having a magnetic field strengths of 3 Tesla or more is used.

Before imaging the first mandrin 200 having the sharp tip 204 is removed from the patient and the hollow needle 100 and an imaging mandrin 300 is introduced into the hollow needle. The imaging mandrin comprises a cylindrical portion 302, a grip 306 located at the proximal end of the cylindrical portion, and a distal end 304 located at the distal end of the cylindrical portion 302. In the interior of the cylindrical portion 302 a cavity 312 may be located which may also be formed cylindrical and extending from the blunt end 304 and the distal end 304, respectively in the direction of the grip 306. The cylindrical portion 302 may be made of plastics. The cavity 312 may be filled by a liquid, for example fish oil, water and/or a contrast agent. The cavity 312 extends as close as possible to the distal end 304, such that during imaging may be evaluated more accurately, where the hollow needle 100 is located in the body of a patient. Liquids, such as water, fish oil and/or contrast agent can be imaged by MR-imaging appropriately, whereas plastics, ceramics, carbon and/or carbon fibers are generally not appropriately imaged by such imaging. By removing the first mandrin 200 from the hollow needle and by introducing the imaging mandrin 300 into the hollow needle before imaging it can be assured that the patient is not impaired by a heated mandrin 200 and that the position of the hollow needle 100 may be located appropriately by virtue of the imaging mandrin 300 introduced therein.

Once the physician has verified the position of the hollow needle 100 in the body of the patient the imaging mandrin 300 may be removed from the hollow needle 100. Thereafter, the first mandrin 200 is again introduced by the grip 206 into the hollow needle and the hollow needle is moved in to the direction of the lesion as soon as the tip 204 projects from the hollow needle 100 at the distal end thereof.

The above described imaging may be repeated as many times as possible until the distal end 304 of the imaging mandrin has reached the lesion. Before each further imaging the first mandrin 200 is removed from the hollow needle 100 and the imaging mandrin 300 is introduced into the hollow needle. It is to be understood that after the imaging the imaging mandrin 300 is removed from the hollow needle 100 and the first mandrin 200 is introduced again into the hollow needle.

Concluding, the application of the present invention in medical diagnostic is described explanatory. The application of the present invention is particularly indicated, if as imaging modality a magnet resonance tomograph having a magnetic field strength of 3 Tesla or more, such as 7 Tesla, is used for the static magnetic field. The present invention may also be used in combination with other imaging modalities, for example with a ultrasonic modality or a computer tomograph modality.

During application of the present invention in combination with a magnet resonance tomograph the patient is lying on the table of the magnet resonance tomograph. Initially the table and the patient are moved into the static magnetic field of the magnet resonance tomograph, which is termed by chargon "to move into the tube". Thereafter, RF-signals and gradient signals are applied and a MR-imaging is executed. By virtue of such imaging the physician may find an appropriate incision location for puncture.

After this first imaging the patient and the patient table are moved out of the static magnetic field and the magnet resonance tomograph, respectively and the physician applies a local anesthesia at the region to be punctured. Further, the physician can apply an incision in the skin of the patient such that the hollow needle 100 and the mandrin may be introduced into the patient more easily. The physician introduces the hollow needle 100 and the mandrin 200 into the body of the patient, wherein the sharp tip 204 of the mandrin projects from the hollow needle.

After a predetermined distance or a distance set by the physician along which the hollow needle 100 and the first mandrin 200 have been introduced into the body of the patient, the physician can execute a further imaging to ensure that the hollow needle 100 and the mandrin have been moved in the appropriate direction. Before executing the imaging, such as before the patient is moved into the static magnetic field of the magnet resonance tomograph and "into the tube" the physician may remove the first mandrin 200 having the sharp tip 204 from the hollow needle 100 and may introduce the imaging mandrin 300 with the fluid filled cavity 312 into the hollow needle. It is to be understood that the physician may remove the first mandrin from the hollow needle 100 and introduce the imaging mandrin 300 into the hollow needle 100 outside the static magnetic field of the magnet resonance tomograph. As soon as the imaging mandrin 300 is inserted into the hollow needle 100 and as soon as the patient is located within the static magnetic field of the magnet resonance tomograph and "within the tube" of the magnet resonance tomograph, a further imaging may be executed by the magnet resonance tomograph. The physician may judge based on the imaging, whether the hollow needle is located at the appropriate location within the body. The physician may remove the imaging mandrin outside the magnet resonance tomograph from the hollow needle 100 and introduce the first mandrin 200 having the sharp tip 204 again into the patient. If necessary, the physician may carry out corrections concerning the position of the hollow needle 100 or move the hollow needle 100 and the mandrin 200 into the direction of the lesion.

It is to be understood that the movement of the hollow needle 100 and of the first mandrin 200 in direction of the lesion and the consecutive imaging may carried out in an arbitrary number until the hollow needle is located at the correct position within the body of the patient. During this procedure the patient on the table of the magnet resonance tomograph may be moved into and out of the uniform static magnetic field of the magnet resonance tomograph, wherein introducing of the hollow needle 100 and of the first mandrin 200 are conducted outside the static uniform magnetic field and imaging is conducted within the static uniform magnetic field.

As soon as the hollow needle 100 is located at the position desired by the physician both the first mandrin 200 and the imaging mandrin 300 are removed from the hollow needle and the actual biopsy needle for conducting tissue removal may be introduced into the hollow needle 100.

The present invention has the advantage that an MR-guided biopsy is provided in which no artifacts in the region of the biopsy needle occur. Further, heating of the biopsy needle and impairment of the comfort of the patient or even a burn are avoided.

## Claims

1. Biopsy needle system (100, 200, 300), having
- a hollow needle (100) having a cylindrical portion (102) adapted to be introduced into a patient, wherein the cylindrical portion comprises a first material;
- a first mandrin (200) comprising a sharp tip (204) and that may be introduced into the hollow needle (100) such that the tip (204) and an opposing end (206) of the first mandrin (200) project from the opposing end of the hollow needle (100), if the hollow needle (100) is located in the patient, wherein the sharp tip (204) is adapted to pierce tissue; and
an imaging mandrin (300) comprising a second material and that may be inserted into the hollow needle (100) if the hollow needle (100) is located in a patient, a distal end (304) and a grip (306) at a proximal end, wherein the second material is a material that may be imaged appropriately during MR-imaging, wherein the distal end (304) of the imaging mandarin (300) is blunt, **characterized in that** the hollow needle is adapted to be positioned at any region to be punctured.

2. Biopsy needle system (100, 200, 300) according to claim 1, wherein the first material is a non ferromagnetic material and/or a non metallic material.

3. Biopsy needle system (100, 200, 300) according to claim 1 or 2, wherein the first material comprises at least one of the following materials:
- plastics;
- carbon;
- carbon fiber; and/or
- ceramics.

4. Biopsy needle system (100, 200, 300) according to one of claims 1 to 3, wherein the cylindrical portion (302) of the imaging mandrin (300) comprises plastics, wherein the cylindrical portion (302) is adapted to be introduced into the hollow needle (100).

5. Biopsy needle system (100, 200, 300) according to one of claims 1 to 4, wherein the imaging mandrin (300) comprises a cavity (312) in which a liquid and/or a gel are located.

6. Biopsy needle system (100, 200, 300) according to claim 5, wherein the fluid and/or the gel comprises at least one of the following fluids:
- oil;
- fish oil;
- water; and/or
- contrast agent.

7. Biopsy needle system (100, 200, 300) according to one of claims 1 to 6, wherein the imaging mandrin (300) does not comprise a sharp tip (304).

## Patentansprüche

1. Biopsienadelsystem (100, 200, 300), mit
- einer Hohlnadel (100) mit einem zylinderförmigen Bereich (102), der dazu ausgebildet ist, in einen Patienten eingeführt zu werden, wobei der zylinderförmige Bereich ein erstes Material aufweist;
- einem ersten Mandrin (200), das eine scharfe Spitze (204) aufweist und das in die Hohlnadel (100) so eingeführt werden kann, dass die Spitze (204) und ein entgegengesetztes Ende (206) des ersten Mandrin (200) aus entgegengesetzten Enden der Hohlnadel (100) herausragen, wenn sich die Hohlnadel (100) in einem Patienten befindet, wobei die scharfe Spitze (204) dazu ausgebildet ist, Gewebe zu stechen;
- ein Bildgebungs-Mandrin (300), das ein zweites Material aufweist und in die Hohlnadel (100) einsetzbar ist, wenn sich die Hohlnadel (100) in einem Patienten befindet, und ein distales Ende 304 und einen Griff (306) aufweisend wobei das zweite Material ein Material ist, das mittels MR-Bildgebung gut abgebildet werden kann, wobei das distale Ende (304) des Bildgebungs-Mandrin (300) stumpf ist, **dadurch gekennzeichnet, dass** die Hohlnadel dazu ausgebildet ist, an einem beliebeigen zu punktierenden Bereich angeordnet zu werden.

2. Biopsienadelsystem (100, 200, 300) nach Anspruch 1, wobei , dass das erste Material ein nicht ferromagnetisches Material und/oder ein nicht metallisches Material ist.

3. Biopsienadelsystem (100, 200, 300) nach Anspruch 1 oder 2, wobei , dass das erste Material zumindest eines der folgenden Materialien aufweist:
- Kunststoff;
- Carbon;
- Kohlefaser; und/oder
- Keramik.

4. Biopsienadelsystem (100, 200, 300) nach einem der Ansprüche 1 bis 3, wobei der zylinderförmiger Bereich (302) des Bildgebungs-Mandrin (300) Kunststoff aufweist, wobei der zylinderförmige Bereich (302) dazu ausgebildet ist, in die Hohlnadel (100) eingeführt zu werden.

5. Biopsienadelsystem (100, 200, 300) nach einem der Ansprüche 1 bis 4, wobei das Bildgebungs-Mandrin (300) einen Hohlraum (312) aufweist, in dem sich eine Flüssigkeit und/oder ein Gel befindet.

6. Biopsienadelsystem (100, 200, 300) nach Anspruch 5, wobei die Flüssigkeit und/oder das Gel zumindest eine der folgenden Flüssigkeiten aufweist:
- Öl;
- Fischöl;
- Wasser; und/oder
- Kontrastmittel.

7. Biopsienadelsystem (100, 200, 300) nach einem der Ansprüche 1 bis 6, **dadurch gekennzeichnet, dass** das Bildgebungs-Mandrin (300) keine scharfe Spitze (304) aufweist.

## Revendications

1. Système d'aiguille de biopsie (100, 200, 300), comportant :
- une aiguille creuse (100) qui comporte une section cylindrique (102) qui est adaptée de manière à ce qu'elle soit introduite à l'intérieur du corps d'un patient, dans lequel la section cylindrique comprend un premier matériau ;
- un premier mandrin (200) qui comprend une pointe acérée (204) et qui peut être introduit à l'intérieur de l'aiguille creuse (100) de telle sorte que la pointe (204) et une extrémité opposée (206) du premier mandrin (200) fassent saillie depuis l'extrémité opposée de l'aiguille creuse (100), si l'aiguille creuse (100) est localisée à l'intérieur du corps du patient, dans lequel la pointe acérée (204) est adaptée de manière à ce qu'elle perce un tissu ; et
un mandrin d'imagerie (300) qui comprend un second matériau et qui peut être inséré à l'intérieur de l'aiguille creuse (100) si l'aiguille creuse (100) est localisée à l'intérieur du corps d'un patient, une extrémité distale (304) et un moyen de préhension (306) au niveau d'une extrémité proximale, dans lequel le second matériau est un matériau qui peut être imagé de manière appropriée pendant une imagerie RM, dans lequel l'extrémité distale (304) du mandrin d'imagerie (300) est émoussée, **caractérisé en ce que** l'aiguille creuse est adaptée de manière à ce qu'elle soit positionnée au niveau de n'importe quelle région qui doit faire l'objet d'une ponction.

2. Système d'aiguille de biopsie (100, 200, 300) selon la revendication 1, dans lequel le premier matériau est un matériau non ferromagnétique et/ou un matériau non métallique.

3. Système d'aiguille de biopsie (100, 200, 300) selon la revendication 1 ou 2, dans lequel le premier matériau comprend au moins l'un des matériaux qui suivent :
- une matière plastique ;
- du carbone ;
- une fibre de carbone ; et/ou
- une céramique.

4. Système d'aiguille de biopsie (100, 200, 300) selon l'une des revendications 1 à 3, dans lequel la section cylindrique (302) du mandrin d'imagerie (300) comprend une matière plastique, dans lequel la section cylindrique (302) est adaptée de manière à ce qu'elle soit introduite à l'intérieur de l'aiguille creuse (100).

5. Système d'aiguille de biopsie (100, 200, 300) selon l'une des revendications 1 à 4, dans lequel le mandrin d'imagerie (300) comprend une cavité (312) à l'intérieur de laquelle un liquide et/ou un gel sont/est localisé(s).

6. Système d'aiguille de biopsie (100, 200, 300) selon la revendication 5, dans lequel le fluide et/ou le gel comprennent/comprend au moins l'un des fluides qui suivent :
- de huile ;
- de huile de poisson ;
- de l'eau ; et/ou
- un agent de contraste.

7. Système d'aiguille de biopsie (100, 200, 300) selon l'une des revendications 1 à 6, dans lequel le mandrin d'imagerie (300) ne comprend pas une pointe acérée (304).
